(19) Europäisches Patentamt
European Patent Office
Office européen des brevets

(11) Publication number: **0 097 480**
**A1**

(12) # EUROPEAN PATENT APPLICATION

(21) Application number: 83303423.4

(22) Date of filing: 14.06.83

(51) Int. Cl.³: C 07 D 401/14
C 07 D 409/14, A 61 K 31/44
A 61 K 31/505, A 01 N 43/64
//(C07D401/14, 249/08, 213/61, 239/30), (C07D401/14, 249/08, 213/61, 257/04), (C07D409/14, 249/08, 213/61, 333/28)

(30) Priority: 17.06.82 GB 8217521

(43) Date of publication of application:
04.01.84 Bulletin 84/1

(84) Designated Contracting States:
AT BE CH DE FR GB IT LI LU NL SE

(71) Applicant: Pfizer Limited
Ramsgate Road
Sandwich Kent CT13 9NJ(GB)

(84) Designated Contracting States:
GB

(71) Applicant: Pfizer Corporation
Calle 15 1/2 Avenida Santa Isabel
Colon(PA)

(84) Designated Contracting States:
BE CH DE FR IT LI LU NL SE AT

(72) Inventor: Richardson, Kenneth, Dr.
48 St. Stephens Hill
Canterbury Kent(GB)

(72) Inventor: Whittle, Peter John, Dr.
5 Winchester Gardens
Canterbury Kent(GB)

(74) Representative: Moore, James William
Pfizer Limited Ramsgate Road
Sandwich Kent CT13 9NJ(GB)

(54) **Triazole antifungal agents.**

(57) 1-[2-Heterocyclyl-2-(5-chloro-2-pyridyl)-2-hydroxyethyl]-1,2,4-triazoles are antifungal agents useful for the treatment of fungal infections in humans and other animals.

EP 0 097 480 A1

COMPLETE DOCUMENT

This invention relates to novel triazole derivatives which have antifungal activity and are useful in the treatment of fungal infections in animals, including humans.

According to the invention, there are provided 1-[2-heterocyclyl-2-(5-chloro-2-pyridyl)-2-hydroxy-ethyl]1,2,4-triazoles of the formula:

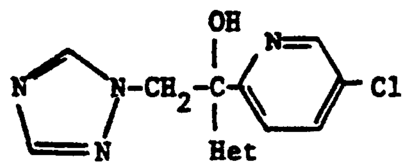 ---- (I)

wherein Het is an aromatic heterocyclic group;
and their pharmaceutically acceptable salts.

The invention also provides a pharmaceutical composition comprising a compound of the formula (I) or a pharmaceutically acceptable salt thereof, together with a pharmaceutically acceptable diluent or carrier.

The invention further provides a compound of the formula (I) or a pharmaceutically acceptable salt thereof, for use in treating fungal infections in animals, including humans.

The term Het includes 5 and 6 membered aromatic heterocyclic ring systems containing up to four heteroatoms selected from N, S or O, which may optionally be fused to a benzene or further heterocyclic ring and which may optionally be substituted by up to three substituents chosen from fluoro, chloro, bromo, iodo, $C_1$-$C_4$ alkyl, $C_1$-$C_4$ alkoxy and $CF_3$, in either the heterocyclic or fused ring.

Particular examples of heterocyclic groups include pyridyl, pyrimidinyl, thienyl, triazolyl and tetrazolyl groups which may optionally be substituted, especially with halo or $C_1$-$C_4$ alkyl groups.

Preferred heterocyclic groups include pyridyl, especially 5-chloro-2-pyridyl, and thienyl, especially 5-chloro-2-thienyl.

Thus particularly preferred individual compounds of the invention include

1-[2,2-Bis(5-chloro-2-pyridyl)-2-hydroxyethyl]1,2,4-triazole and

1-[2-(5-chloro-2-pyridyl)-2-(5-chloro-2-thienyl)-2-hydroxyethyl]1,2,4-triazole.

The compounds of formula (I) can be prepared by reacting an oxirane of formula II with triazole according to the following reaction scheme:

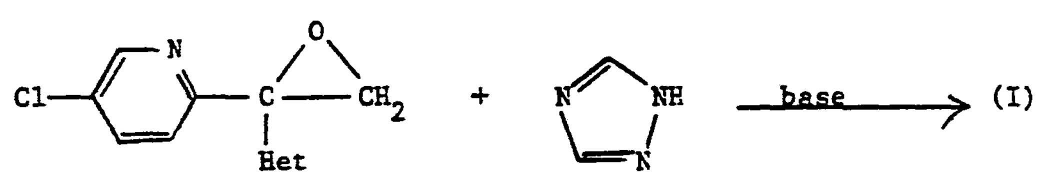


(II)

The reaction is generally achieved with the reactants dissolved in a reaction-inert organic solvent, e.g. N,N-dimethylformamide, in the presence of a base, e.g. potassium carbonate. The reaction can be accelerated by heating and we have found that a period of 2 hours at 80 to 90°C is generally sufficient to ensure that the reaction is substantially complete.

The product is isolated by evaporation of the solvent followed by extraction with an organic solvent, e.g. ethyl acetate or methylene chloride, to separate the product from the inorganic solids. The crude product may be further purified, if desired, by conventional procedures, for example using chromatography or by recrystallisation from a suitable solvent.

The starting materials of formula (II) can be obtained by conventional methods from the appropriate heterocyclic compounds. In a typical case they are prepared by reacting a halo-substituted derivative (III) of the desired heterocyclic compound with butyl lithium to generate the anion, which is then reacted with the amide (IV) prepared for example from 2-bromo-5-chloropyridine and dimethylcarbamoyl chloride, and the product (V) is converted to the oxirane (II). The route is shown in the following reaction scheme wherein X is chloro, bromo or iodo.

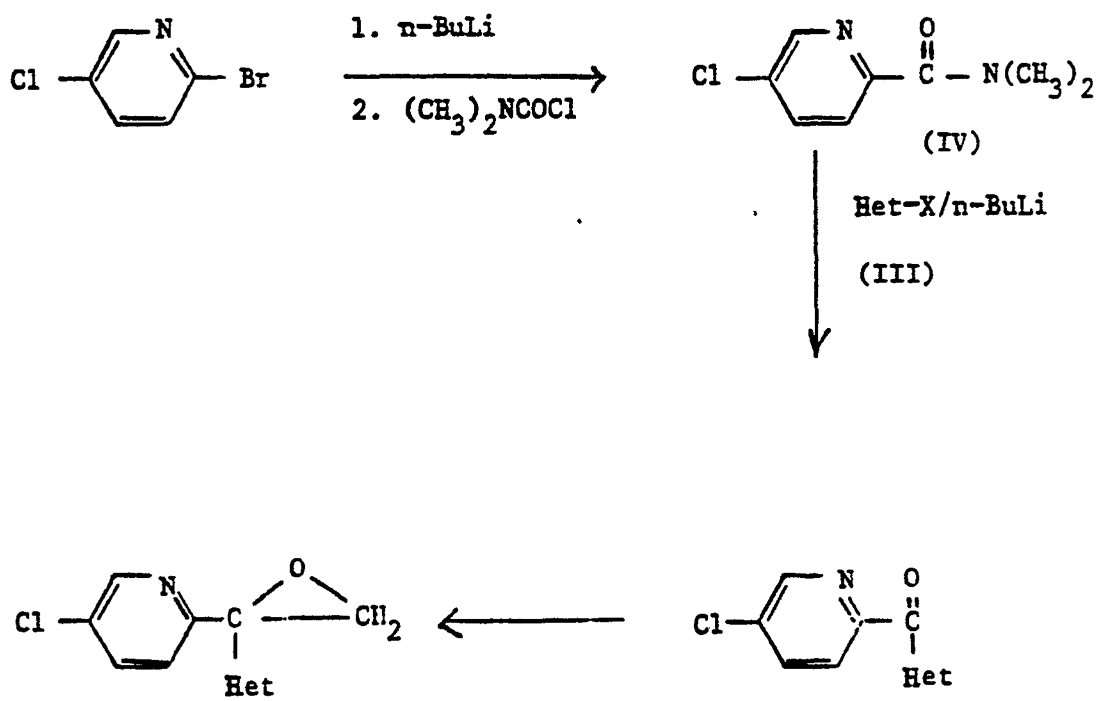


(II) (V)

The halo-substituted heterocyclic compounds (III), preferably the bromo derivatives, are generally known compounds which are either commercially available or they are prepared by conventional methods in accordance with literature precedents. In certain instances the required anion can be generated directly from the heterocyclic compound e.g. by reaction with lithium diisopropylamide.

The amide (IV) is simply prepared from 2-bromo-5-chloropyridine by reaction first with n-butyl lithium to generate the anion, followed by reaction with dimethylcarbamoyl chloride or tetramethylurea. The reaction is typically achieved in diethyl ether at -70°C.

The ketones (V) are then prepared by reacting the amide (IV) with the anion derived from the appropriate halo-substituted heterocyclic derivative (III).

In the case of the compound wherein het is 5-chloro-2-pyridyl these two steps can be combined by using a 2-fold excess of 2-bromo-5-chloropyridine in the reaction with dimethylcarbamoyl chloride, and the ketone (V) wherein het is 5-chloro-2-pyridyl can be isolated directly.

The oxiranes (II) can be obtained from the ketones (V) by reaction with dimethyloxosulphonium methylide prepared from trimethylsulphoxonium iodide and either sodium hydride in dimethylsulphoxide or using cetrimide and sodium hydroxide in a mixture of water and toluene.

The reaction using sodium hydride is typically achieved by adding dry powdered trimethylsulphoxonium iodide to a suspension of sodium hydride in dimethylsulphoxide. After stirring for, say,

30 minutes at room temperature, the ketone (V) is added in an approximately equimolar amount in dimethylsulphoxide. The reaction mixture may be warmed to accelerate the reaction and after several hours at 50°-80°C, the product can be isolated by conventional procedures.

The reaction utilising cetrimide is typically achieved by stirring the ketone (V), trimethylsulphoxonium iodide and cetrimide vigorously together in a mixture of toluene or trichloroethane and sodium hydroxide solution for about an hour at up to about 100°C. The oxirane product can then be isolated by conventional procedures.

In an alternative procedure the oxirane can in some cases be more readily obtained by reacting the anion derived from 2-bromo-5-chloropyridine directly with a bromoacetyl-heterocyclic compound.

It is not generally necessary to isolate the product (II) but it can be reacted directly with triazole as previously described to give the compound of formula (I).

Pharmaceutically acceptable acid addition salts of the compounds of the formula (I) include those formed from strong acids which form non-toxic acid addition salts, such as hydrochloric, hydrobromic, sulphuric, nitric, oxalic and methane sulphonic acids.

The salts may be obtained by conventional procedures, e.g. by mixing solutions containing equimolar amounts of the free base and desired acid, and the required salt is collected by filtration, if insoluble, or by evaporation of the solvent.

The compounds of the formula (I) and their pharmaceutically acceptable salts are anti-fungal agents, useful in combating fungal infections in animals, including humans. For example they are useful in treating topical fungal infections in man caused by, among other organisms, species of Candida, Trichophyton, Microsporum, or Epidermophyton, or in mucosal infections caused by Candida albicans (e.g. thrush and vaginal candidiasis). They may also be used in the treatment of systemic fungal infections caused by, for example, Candida albicans, Cryptococcus neoformans, Aspergillus fumigatus, Coccidioides, Paracoccidioides, Histoplasma or Blastomyces.

The in vitro evaluation of the anti-fungal activity of the compounds can be performed by determining the minimum inhibitory concentration (m.i.c.) of the test compounds in a suitable medium at which growth of the particular micro-organism fails to occur. In practice, a series of agar plates, each having the test compound incorporated at a particular concentration are inoculated with a standard culture of, for example, Candida albicans and each plate is then incubated for 48 hours at 37°C. The plates are then examined for the presence or absence of growth of the fungus and the appropriate m.i.c. value is noted. Other micro-organisms used in such tests can include Cryptococcus neoformans, Aspergillus fumigatus, Trichophyton spp; Microsporum spp; Epidermophyton floccosum, Coccidioides immitis, and Torulopsis glabrata.

The in vivo evaluation of the compounds can be carried out at a series of dose levels by intraperitoneal or intravenous injection or by oral administration, to mice which are inoculated with a strain of Candida albicans. Activity is based on the

survival of a treated group of mice after the death of an untreated group of mice following 48 hours observation. The dose level at which the compound provides 50% protection against the lethal effect of the infection is noted.

For human use, the anti-fungal compounds of the formula (I) can be administered alone, but will generally be administered in admixture with a pharmaceutical carrier selected with regard to the intended route of administration and standard pharmaceutical practice. For example, they may be administered orally in the form of tablets containing such excipients as starch or lactose, or in capsules or ovules either alone or in admixture with excipients, or in the form of elixirs or suspensions containing flavouring or colouring agents. They may be injected parenterally, for example, intravenously, intramuscularly or subcutaneously. For parenteral administration, they are best used in the form of a sterile aqueous solution which may contain other substances, for example, enough salts or glucose to make the solution isotonic with blood.

For oral and parenteral administration to human patients, it is expected that the daily dosage level of the anti-fungal compounds of the formula (I) will be from 0.1 to 10 mg/kg (in divided doses) when administered by either the oral or parenteral route. Thus tablets or capsules of the compounds can be expected to contain from 5 mg to 0.5 g of active compound for administration singly or two or more at a time as appropriate. The physician in any event will determine the actual dosage which will be most suitable for an individual patient and it will vary with the age, weight and response of the particular patient. The

above dosages are exemplary of the average case; there can, of course, be individual instances where higher or lower dosage ranges are merited, and such are within the scope of this invention.

Alternatively, the anti-fungal compounds of formula (I) may be administered in the form of a suppository or pessary, or they may be applied topically in the form of a lotion, solution, cream, ointment or dusting powder. For example, they may be incorporated into a cream consisting of an aqueous emulsion of polyethylene glycols or liquid paraffin; or they may be incorporated, at a concentration between 1 and 10%, into an ointment consisting of a white wax or white soft paraffin base together with such stabilizers and preservatives as may be required.

The compounds of the formula (I) and their salts also have activity against a variety of plant pathogenic fungi, including for example various rusts, mildews and moulds, and the compounds are thus useful as agricultural fungicides for treating plants and seeds to eradicate or prevent such diseases.

The following Examples illustrate the invention:-

EXAMPLE 1

1-[2,2-Bis-(5-chloro-2-pyridyl)-2-hydroxyethyl]1,2,4-triazole

2,2-Bis(5-chloro-2-pyridyl)oxirane (0.8 g, the crude product from Preparation 4), 1,2,4-triazole (1.0 g) and anhydrous potassium carbonate (3.0 g) were stirred in dry N,N-dimethylformamide (30 ml) and the mixture heated at 85°C for 1½ hours. The solvent was then evaporated under vacuum, with the addition of xylene to remove final traces of N,N-dimethylformamide as an azeotrope, and the residue was dissolved in water (20 ml) and extracted with ethyl acetate (3 x 20 ml). The combined organic extracts were dried over $MgSO_4$ and evaporated. The residue was chromatographed on silica, eluting with ethyl acetate. Evaporation of the relevant fractions gave a viscous oil which solidified on trituration with petroleum ether (b.p. 60-80°C). Recrystallisation from hexane gave the title product as white crystals (0.48 g, 38%), m.p. 98-100°C.

Analysis %:-

| | |
|---|---|
| Found: | C,50.0; H,3.3; N,21.6 |
| $C_{14}H_{11}Cl_2N_5O$ requires: | C,50.0; H,3.3; N,20.8. |

M/e 336 (M + 1)

## EXAMPLES 2-5

The following compounds of formula I were prepared according to the procedure of Example 1, starting with the appropriate oxirane of formula II.

| Example No. | Het | m.p. °C | Analysis % (Theoretical in brackets) C | H | N |
|---|---|---|---|---|---|
| 2 | 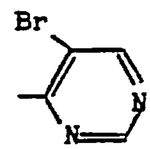 | 120-121 | 41.0<br>(40.9 | 2.8<br>2.6 | 22.3<br>22.0) |
| 3 | 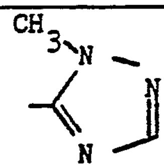 | 178-180 | 47.1<br>(47.1 | 4.1<br>4.0 | 32.2<br>32.1) |
| 4 | 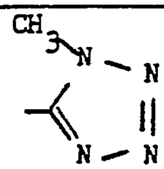 | 168-170 | 42.8<br>(43.1 | 3.6<br>3.6 | 36.6<br>36.5) |
| 5 | 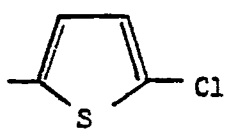 | 145-146 | 45.5<br>(45.8 | 2.9<br>3.0 | 16.7<br>16.4) |

## EXAMPLE 6

The following illustrate pharmaceutical compositions for the treatment of fungal infections:

(1) Capsule: 71 parts by weight of the compound of Example 1 are granulated with 3 parts maize starch and 22 parts lactose and then

a further 3 parts of maize starch and 1 part magnesium stearate are added. The mixture is regranulated and filled into hard gelatin capsules.

(2) Cream: 2 parts by weight of the compound of Example 1 are dissolved in 10 parts of propylene glycol and mixed into 88 parts of a vanishing cream base.

(3) Pessary: 2 parts by weight of the compound of Example 1 are suspended in 98 parts of a warm liquified suppository base which is poured into moulds and allowed to solidify.

## PREPARATION 1

5-Chloro-2-N,N-dimethylcarbamoyl-pyridine

A solution of n-butyllithium in hexane (100 ml, 1.55 molar) was added under nitrogen to a stirred solution of 2-bromo-5-chloropyridine (29.4 g) in dry hexane (180 ml) at -78°C over a period of one hour. The mixture was stirred for a further 20 minutes at -78°C and then a solution of tetramethylurea (20 g, 0.17 mole) in dry diethyl ether (60 ml) was added over 15 minutes. The mixture was stirred at a temperature between -70° and -60°C for a further two hours and then allowed to warm to 0°C. A solution of ammonium chloride (60 g) in water (400 ml) was added and the organic phase separated. The aqueous phase was evaporated to dryness and the residue extracted with hot ethyl acetate (3 x 500 ml). The combined organic phase and organic extracts were dried over $MgSO_4$ and evaporated to yield a dark oil which was distilled under vacuum. The fraction boiling between 100 and

114°C at 0.3 mm mercury was collected (20 g) and further purified by chromatography on silica eluting with ethyl acetate and finally recrystallised from cyclohexane to yield the desired product (10 g), m.p. 50-53°C. Found: C,51.8; H,5.0; N,14.8. $C_8H_9ClN_2O$ requires C,52.0; H,4.9; N,15.2%.

PREPARATION 2

4-[(5-Chloro-2-pyridyl)carbonyl]-5-bromopyrimidine

A solution of n-butyllithium in hexane (5 ml, 1.55 molar) was added to a solution of dry diisopropylamine (0.84 g, 8.3 mmole) in dry diethyl ether (8 ml) at a temperature of from -10° to -15°C. The mixture was stirred for 30 minutes and then cooled to -78°C and a solution of 5-bromopyrimidine (1.44 g, 9 mmole) in dry diethylether was added over 15 minutes. The solution was stirred for 30 minutes and a solution of 5-chloro-2-N,N-dimethylcarbamoyl-pyridine (1.33 g, 7.2 mmole) in dry diethyl ether (20 ml) was then added over 15 minutes. The solution was stirred for a further hour at -78°C and then allowed to warm to 0°C over 1½ hours. Acetic acid (4 ml) was added followed by water (20 ml). The aqueous phase was separated and extracted with diethyl ether (2 x 50 ml). The combined organic phase and ether extracts were washed with dilute aqueous sodium carbonate solution until the aqueous phase remained neutral and the organic layer was then dried over $MgSO_4$ and evaporated. The crude product was chromatographed on silica, eluting with a 3:1 mixture of petroleum ether (b.p. 60-80°C) and ethyl acetate and the product

recrystallised from ethyl acetate to yield the title compound (0.28 g), m.p. 156-160°C. Found: C,40.4; H,1.8; N,14.4. $C_{10}H_5ClN_3O$ requires C,40.2; H,1.7; N,14.1%.

The following ketones were prepared in a similar manner but starting with 1-methyl-1,2,3,4-tetrazole or 1-methyl-1,2,4-triazole respectively:

1-methyl-5-[(5-chloro-2-pyridyl)carbonyl]-tetrazole.
m.p. 140-143°C

1-methyl-5-[(5-chloro-2-pyridyl)carbonyl]-1,2,4-triazole.
m.p. 133-135°C.

PREPARATION 3

Bis-(5-chloro-2-pyridyl)ketone

A solution of n-butyllithium in hexane (16.3 ml, 1.55 molar) was added under nitrogen to a stirred solution of 2-bromo-5-chloropyridine (5 g) in dry diethyl ether (70 ml) at -70°C over a period of 20 minutes. A solution of dimethylcarbamoylchloride (1.36 g) in dry diethylether (25 ml) was then added slowly over a period of 20 minutes. The mixture was stirred for a further 20 minutes at -70°C and a solution of ammonium chloride (5 g) in water (50 ml) was then added and the solution allowed to warm to room temperature. The ether layer was separated and the aqueous solution extracted with diethyl ether (2 x 50 ml). The ether extracts were combined, dried over $MgSO_4$ and evaporated. The residue was chromatographed on silica, eluting with a mixture of ethyl acetate and petroleum ether b.p. 60-80°C (1:9). The

relevant fractions were combined and evaporated and the residue triturated with diethyl ether and dried to yield the title compound as a buff coloured solid (0.86 g). Found: C,52.4; H,2.5; N,11.0. $C_{11}H_6Cl_2NO$ requires C,52.2; H,2.3; N,11.1.

T.L.C. Rf (Silica; ethyl acetate:petroleum ether 3:7) 0.34
m/e 252

PREPARATION 4

Preparation of 2,2-Bis-(5-chloro-2-pyridyl)oxirane

A mixture of bis-(5-chloro-2-pyridyl)ketone (0.8 g), trimethylsulphoxonium iodide (0.84 g), cetrimide (0.08 g) and sodium hydroxide (3 g) in water (16 ml) and 1,1,1-trichloroethane (30 ml) was stirred vigorously and heated at 70 to 75°C for 2½ hours. Further trimethylsulphoxonium iodide (0.86 g) was added and the mixture heated at 75 to 80°C for a further 2 hours. The mixture was cooled, diluted with water (10 ml) and extracted with dichloromethane (3 x 20 ml). The combined organic extracts were dried over $MgSO_4$ and evaporated to yield the crude product as a brownish paste (0.8 g), which was used directly in Example 1.

T.L.C. Rf (silica, $CH_2Cl_2$) 0.20

The ketones from Preparation 2 were converted to the corresponding oxirane derivatives in a similar manner to the above and used directly as the starting materials for Examples 2 to 4.

## PREPARATION 5

2-(5-Chloro-2-pyridyl)-2-(5-chloro-2-thienyl)oxirane

A solution of n-butyllithium in hexane (9.6 ml, 1.55 molar) was added under nitrogen to a stirred solution of 2-bromo-5-chloropyridine (3 g) in dry diethyl ether (50 ml) at -70°C over a period of 25 minutes. The solution was stirred for a further 20 minutes at -70°C and a solution of 2-bromoacetyl-5-chloro-thiophene (3 g) in dry diethyl ether (50 ml) was then added over 20 minutes. The mixture was stirred at -70°C for a further hour and then allowed to warm to -40°C over a further hour. Acetic acid (3 ml) and water (20 ml) were added and the solution allowed to warm to room temperature. The organic layer was separated, the aqueous layer washed with diethyl ether (2 x 50 ml), and the combined organic layers were washed with water (20 ml), dried over $MgSO_4$ and evaporated to yield the crude oxirane as a dark red oil.

The product was used directly as the starting material for Example 5.

CLAIMS

1. A compound of the formula:

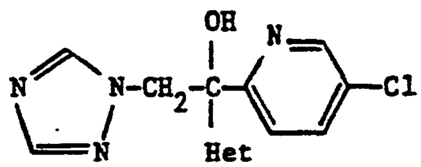

--- (I)

wherein Het is an aromatic heterocyclic group;
and their pharmaceutically acceptable salts.

2. A compound according to claim 1 wherein Het is a 5 or 6 membered aromatic heterocyclic ring system containing up to four heteroatoms selected from N, S or O, which may optionally be fused to a benzene or further heterocyclic ring and which may optionally be substituted by up to three substituents chosen from fluoro, chloro, bromo, iodo, $C_1$-$C_4$ alkyl, $C_1$-$C_4$ alkoxy and $CF_3$, in either the heterocyclic or fused ring.

3. A compound according to claim 2 wherein Het is a pyridyl, pyrimidinyl, thienyl, triazolyl or tetrazolyl group which may optionally be substituted with halo or $C_1$-$C_4$ alkyl groups.

4. A compound according to claim 3 wherein Het is a 5-chloro-2-pyridyl or 5-chloro-2-thienyl group.

5. A process for preparing a compound of the formula I as claimed in claim 1 which comprises reacting an oxirane of the formula:

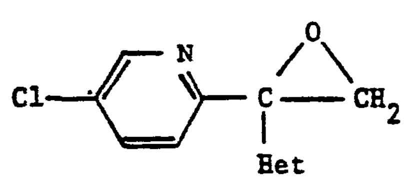

--- (II)

wherein Het is as defined in claim 1, with triazole in an organic solvent in the presence of a base.

6. A pharmaceutical composition comprising a compound of the formula (I) as claimed in any one of claims 1 to 4 or a pharmaceutically acceptable salt thereof, together with a pharmaceutically acceptable diluent or carrier.

7. A compound of the formula (I) as claimed in any one of claims 1 to 4 or a pharmaceutically acceptable salt thereof, for use in treating fungal infections in animals, including humans.

0097480

CLAIMS FOR THE CONTRACTING - STATE AT

1. A process for preparing a compound of the formula:

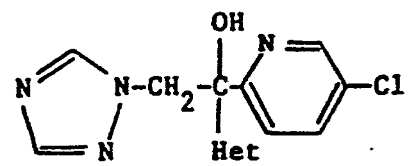

--- (I)

wherein Het is an aromatic heterocyclic group;

which comprises reacting a compound of the formula:

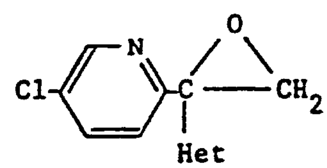

--- (II)

with triazole in an organic solvent in the presence of a base.

2. A process as claimed in claim 1 when performed with the reactants disolved in N,N-dimethylformamide in the presence of potassium carbonate.

3. A process as claimed in claim 1 or claim 2 wherein Het is a 5 or 6 membered aromatic heterocyclic ring system containing up to four heteroatoms selected from N, S or O, which may optionally be fused to a benzene or further heterocyclic ring and which may optionally be substituted by up to three substituents chosen from fluoro, chloro, bromo, iodo, $C_1$-$C_4$ alkyl, $C_1$-$C_4$ alkoxy and $CF_3$, in either the heterocyclic or fused ring.

4. A process as claimed in claim 3 wherein Het is a pyridyl, pyrimidinyl, thienyl, triazolyl or tetrazolyl group which may optionally be substituted with halo or $C_1$-$C_4$ alkyl groups.

5. A process as claimed in claim 4 wherein Het is a 5-chloro-2-pyridyl or 5-chloro-2-thienyl group.

European Patent Office

# EUROPEAN SEARCH REPORT

Application number 0097480

EP 83303423.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | EP - A2 - 0 046 337 (IMPERIAL CHEM. IND.)<br>* Formula I; page 12, line 12 *<br>-- | 1,5 | C 07 D 401/14<br>C 07 D 409/14<br>A 61 K 31/44<br>A 61 K 31/505<br>A 01 N 43/64//<br>(C 07 D 401/14<br>C 07 D 249/08<br>C 07 D 213/61<br>C 07 D 239/30)<br>(C 07 D 401/14<br>C 07 D 249/08<br>C 07 D 213/61<br>C 07 D 257/04)<br>(C 07 D 409/14<br>C 07 D 249/08<br>C 07 D 213/61<br>C 07 D 333/28) |
| A | CHEMICAL ABSTRACTS, vol. 94, no. 9, March 2, 1981, Columbus, Ohio, USA<br>SUMITOMO, "Thiophene and furan compounds"<br>page 707, columns 1,2; abstract--no. 65 464q<br>& Jpn. Kokai Tokkyo 8 094,384, July 17, 1980<br>-- | 1 | |
| A | CHEMICAL ABSTRACT, vol. 94, no. 19, May 11, 1981, Columbus, Ohio, USA<br>SUMITOMO, "1-(2-Furyl)- or 1-(2--Thienyl)-4,4-dimethyl-2-(tri-or diazol-1-yl)-1-penten-3-ones (or 3-ols) as agricultural fungicides"<br>page 662, column 2 - page 663, column 1; abstract-no. 156 932g<br>& Jpn. Kokai Tokkyo 8 087,787, July 2, 1980<br>-- | 1 | TECHNICAL FIELDS SEARCHED (Int. Cl. 3)<br>C 07 D 401/00<br>C 07 D 409/00 |
| A | US - A - 3 558 642 (HARTLEY)<br>* Formula I *<br>---- | 1 | |

The present search report has been drawn up for all claims

| Place of search | Date of completion of the search | Examiner |
|---|---|---|
| VIENNA | 12-08-1983 | HAMMER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document